# EUROPEAN PATENT SPECIFICATION

(11) **EP 2 865 414 B1**
(45) Date of publication and mention of the grant of the patent: **29.07.2020**
(21) Application number: 14190334.4
(22) Date of filing: 24.10.2014
(51) Int. Cl.: A61N 1/375, A61N 1/05, A61B 5/042

(54) **Connection of electrodes to wires coiled on a core**
Verbindung von Elektroden an auf einen Kern gewickelte Drähte
Connexion d'électrodes à des fils enroulés sur un noyau

(30) Priority: 25.10.2013 US 201314063477
(43) Date of publication of application: 29.04.2015
(73) Proprietor: Biosense Webster (Israel) Ltd., Yokneam, 2066717 (IL)
(72) Inventor: Govari, Assaf, 344001 Haifa (IL)
(74) Representative: Carpmaels & Ransford LLP

(56) References cited:
- EP-A1- 1 723 981
- EP-A1- 2 471 480
- US-A1- 2012 271 385

## Description

### FIELD OF THE INVENTION

The present invention relates generally to cabling, and specifically to connection of an electrode to the cabling.

### BACKGROUND OF THE INVENTION

U.S. Patent Application 2012/0172714, to Govari et al., describes a method for incorporating a conducting wire into a tubular braid consisting of a multiplicity of supporting wires, and covering the tubular braid with a sheath. The method further includes identifying a location of the conducting wire within the tubular braid and attaching an electrode through the sheath to the conducting wire at the location.

U. S. Patent 6,213,995, to Steen, et al., describes a flexible tubing which includes a wall provided with a plurality of braided elements forming a braid within the wall of the tube. The braided elements are stated to include one or more signal transmitting elements and one or more metallic or non-metallic structural elements having structural properties different from the signal transmitting elements.

U. S. Patent 7,229,437, to Johnson, et al., describes a catheter having electrically conductive traces and external electrical contacts. The disclosure states that each trace may be in electrical connection with one or more external electrical contacts.

Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

US2012/0271385 discloses techniques related to wires that may be used within a medical device. In one example, the exposed end of the filar may be abutted next to an inner surface of a respective conducting electrode. This exposed portion of the filar may be heated by a resistive-, spot-, or a laser-welding process. This will bond the exposed portion of the filar to this surface of the electrode, forming an electrical and mechanical connection. This bonding may be further strengthened by melting beads of a fusible metal alloy to form electrically conductive joints.

EP2471480 discloses a method, including incorporating a conducting wire into a tubular braid consisting of a multiplicity of supporting wires, and covering the tubular braid with a sheath. The method further includes identifying a location of the conducting wire within the tubular braid and attaching an electrode through the sheath to the conducting wire at the location.

EP1723981 discloses a bi-directional electrophysiology catheter having improved steerability which includes orientation sheaths, or thin walled tubes, placed in diametrically opposed lumen at the distal portion of the catheter for producing in-plane deflection of the distal portion of the catheter.

### SUMMARY OF THE INVENTION

The present invention provides a method for attaching an electrode to cabling, comprising providing a cable comprising a plurality of insulated wires coiled around a central core, wherein the wires are covered with a sheath, and wherein the sheath is transparent, and an insulating layer of at least one of the plurality of insulated wires is colored differently from the colors of insulating layers of the remaining plurality of insulated wires; selecting a location for attaching a respective electrode by finding a colored insulated wire visually from the plurality of insulated wires, and then finding a required location for the respective electrode along the cabling; removing a section of the sheath and removing a corresponding section of insulation from the colored insulated wire in a set of the coiled wires, wherein the section of the sheath is removed from above the wire, so as to provide a respective access channel through the sheath and through the insulation to a respective section of a respective conductor of the wire in the set while the respective section remains coiled on the central core; and fastening the respective electrode to the respective access channel while the respective section remains coiled on the central core, wherein the method further comprises positioning a solder ball in proximity to the respective access channel prior to fastening the respective electrode, and wherein fastening the respective electrode comprises melting the solder ball so that solidified solder connects the respective section of the respective conductor, via the respective access channel, to the respective electrode.

Typically, positioning the solder ball includes applying glue over the respective access channel, and holding the solder ball in place with the glue.

In a further disclosed embodiment the respective electrode completely encircles the cabling. Alternatively, the respective electrode partially encircles the cabling.

In a yet further disclosed embodiment the wires are coiled single-handedly around the central core. Alternatively, the set includes a first group of the wires coiled left-handedly around the central core, and a

second group of the wires coiled right-handedly around the central core.

The cabling may be used in an invasive medical procedure.

Further described herein is an apparatus, including:
a cable consisting of a plurality of insulated wires coiled around a central core;
a respective access channel to a respective section of a respective conductor of each wire in a set of the coiled wires, formed by removing insulation from each wire in the set while the respective section remains coiled on the central core; and
a respective electrode fastened to the respective access channel while the respective section remains coiled on the central core.

The present disclosure will be more fully understood from the following detailed description of the embodiments thereof, taken together with the drawings, in which:

### BRIEF DESCRIPTION OF THE DRAWINGS

Figs. 1A and 1B are schematic views of cabling, according to an embodiment of the present invention;
Figs. 2A - 2E are schematic diagrams illustrating steps in attaching an electrode to the cabling, according to embodiments of the present invention;
Fig. 3 is a flowchart describing the steps, according to an embodiment of the present invention; and
Fig. 4 is a schematic illustration of an invasive medical procedure using the cabling, according to an embodiment of the present invention.

### DETAILED DESCRIPTION OF EMBODIMENTS

### OVERVIEW

An embodiment of the present invention provides a method for attaching an electrode to cabling. The cabling comprises a cable having a plurality of insulated wires coiled around a central core. In order to attach the electrode to the cabling, insulation is removed from a section of a given wire, so as to provide an access channel to a corresponding section of a conductor in the given wire. The removal of the insulation is performed while the given wire remains coiled around the central core.

Once the access channel has been formed, the electrode may be fastened to it, while the given wire remains coiled around the central core.

Typically, prior to fastening the electrode, a solder ball is positioned in proximity to the access channel, and may be held in place by glue applied over the access channel. The electrode may then be placed over the solder ball. The fastening then includes electrically connecting the electrode to the conductor by melting, then cooling, the solder so that solidified solder connects the electrode and the corresponding section of the conductor via the access channel. The melting may be implemented by applying heat to the electrode.

### SYSTEM DESCRIPTION

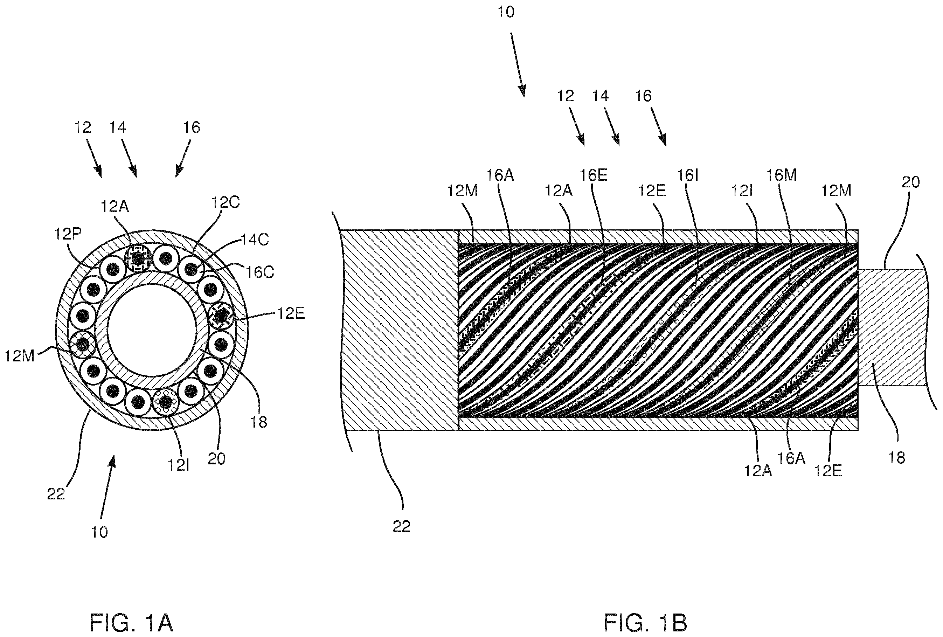

Reference is now made to Figs. 1A and 1B, which are schematic views of cabling 10, according to an embodiment of the present invention. Fig. 1A is a schematic cross-sectional view of the cabling. Fig. 1B is a schematic, side view of cabling 10, which has been partially cut-away to expose internal elements of the cabling. As is explained further below, an electrode is attached to the cabling, and the cabling is typically configured so that a large number of separate electrodes, each having respective attached wires, may be attached in a small length of the cabling, so that cabling 10 is capable of supporting a high density of electrodes. Cabling 10 is typically used as part of a medical catheter wherein electrical measurements are to be made from the electrodes attached to the cabling.

Cabling 10 comprises a plurality of generally similar wires 12, each wire 12 being formed as a conductor 14 covered by an insulating layer 16. In the following description, generally similar components associated with cabling 10 are referred to generically by their identifying component numeral, and are differentiated from each other, as necessary, by appending a letter A, B, ... to the numeral. Thus wire 12C is formed as conductor 14C covered by insulating layer 16C. While embodiments of the present invention may be implemented with substantially any plurality of wires 12 in the cabling, for clarity and simplicity in the following description cabling 10 is assumed to comprise 16 wires 12A, ... 12E, ... 121, ... 12M, ... 12P.

(For purposes of illustration, insulating layers 16 of wires 12 have been drawn as having approximately the same dimensions as conductors 14. In practice, the insulating layer is typically approximately one-tenth the diameter of the wire.)

In order to be used as part of a medical catheter, an outer diameter of cabling 10 is implemented to be as small as possible. In one embodiment, cabling 10 is approximately cylindrical with a length of approximately 2 m, and an outer diameter approximately equal to 0.5 mm.

Wires 12 may be formed with any diameter wire that is consistent with the outer diameter of cabling 10. In one embodiment wires 12 are formed of 48 AWG wire, corresponding to a wire diameter of approximately 30 microns. In some embodiments of the present invention the inventors have used, *inter alia,* monel, constantan, or copper for conductors 14. While copper has a higher electrical conductivity than monel or constantan, it may have a tendency to break during production of the cabling. Both monel and constantan enhance the strength of cabling 10, but in environments where magnetic properties of materials are significant, such as during a magnetic resonance imaging procedure or in a catheter using magnetic navigation, it may be preferable to use constantan for conductors 14.

While monel, constantan and copper are provided as examples of the material used for conductors 14, it will be understood that embodiments of the present invention are not limited to a particular type of material, and any other convenient electrically conducting material may be used. In some embodiments wires that neighbor each other may be selected to have dissimilar conductors, such as copper and constantan, so as to be available for forming a thermocouple junction.

Wires 12 are formed over an internal core 18, which is typically shaped as a cylindrical tube, and core 18 is also referred to herein as tube 18. The core material is typically selected to be a thermoplastic elastomer such as a polyether block amide (PEBA). In a disclosed embodiment core 18 is formed of 40D Pebax, produced by Arkema, Colombes, France. In the disclosed embodiment core 18, by way of example, is cylindrical with a wall thickness of approximately 13 microns, and an outer diameter of approximately 0.4 mm. Wires 12 are formed on an outer surface 20 of core 18 by coiling the wires around the tube. Thus, in the case that core 18 is cylindrical, each wire 12 on the outer surface is in the form of a helical coil. In contrast to a braid, all helical coils of wires 12 have the same handedness, i.e., all wires of the cabling are left-handed, or all wires of the cabling are right-handed. In the present disclosure and in the claims, cabling, wherein all the wires of the cabling that are coiled around an internal core of the cabling have the same handedness, is referred to as single-handed cabling. Also in the present disclosure and in the claims, cabling wherein the wires of the cabling comprise a first group of wires coiled left-handedly and a second group of wires coiled right-handedly, is referred to as braided cabling. Typically in braided cabling the number of wires in the first group equals the number in the second group.

Further details of a single-handed cabling generally similar to cabling 10 are provided in U.S. Patent Application Number 13/860,921, referenced above. An example of a braided cabling is provided in U.S. Patent Application 2012/0172714, referenced above.

For simplicity, the following description considers only single-handed cabling, as is exemplified by cabling 10. However, embodiments of the present invention apply to either single-handed or braided cabling, and those having ordinary skill in the art will be able to adapt the description, *mutatis mutandis,* for braided cabling.

In coiling wires 12 on surface 20, the wires are typically arranged so that they contact each other in a "close-packed" configuration. In other words, if internal tube 18 were to be opened so that surface 20 is a flat plane, the wires (neglecting "end-effects") form a single wire layer over surface 20, with insulating layers 16 continuously in contact with two other insulating layers, and with the insulating layers being continuously in contact with surface 20.

In the case of tube 18 being cylindrical, the close-packed arrangement of the helical coils of wires 12 means that the wires are configured in a multi-start thread configuration. Such a configuration is described in more detail in U.S. Patent Application Number 13/860,921 referenced above.

Once wires 12 have been formed in the multi-start thread configuration described above, the wires are covered with a protective sheath 22. The protective sheath material is typically selected to be a thermoplastic elastomer such as PEBA. In a disclosed embodiment sheath 22 is formed of 55D Pebax, produced by Arkema, and no additives are incorporated in the sheath, so that it is transparent. In the disclosed embodiment sheath 22, by way of example, has an outer diameter of approximately 0.5 mm.

The insulating layer of at least one of wires 12 is colored differently from the colors of the remaining wires. Such coloration aids in identifying particular wires once they have been arranged within cabling 10, assuming that sheath 22 is transparent. An alternative method for identifying particular wires in a braided arrangement is described in U.S. Patent Application 2012/0172714.

By way of example, in the embodiment described herein, using 16 wires, every fourth wire has its insulating layer colored, so that insulating layers 16A, 16E, 161, and 16M are respectively colored green, black, red, and violet. The insulating layers of the remaining wires may be given another color, such as white, or may be left colorless. Thus, wires 12A, 12E, 121, and 12M appear to have the colors green, black, red, and violet, and are visually different in appearance from the remaining wires.

The process of coiling wires 12 around a core, and then covering the wires by a sheath, essentially embeds the wires within a wall of cabling 10, the wall consisting of the core and the sheath. Embedding the wires within a wall means that the wires are not subject to mechanical damage when the cabling is used to form a catheter. Mechanical damage is prevalent for small wires, such as the 48 AWG wires exemplified above, if the wires are left loose during assembly of a catheter.

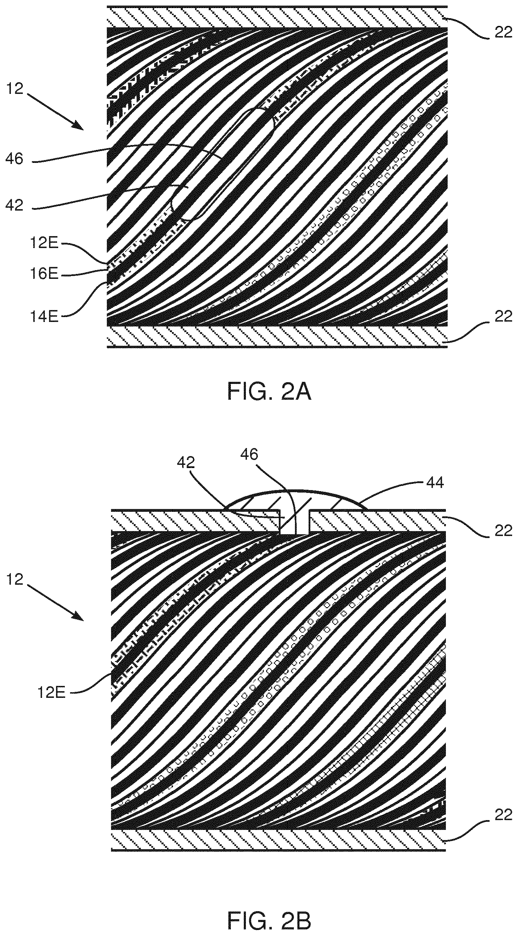

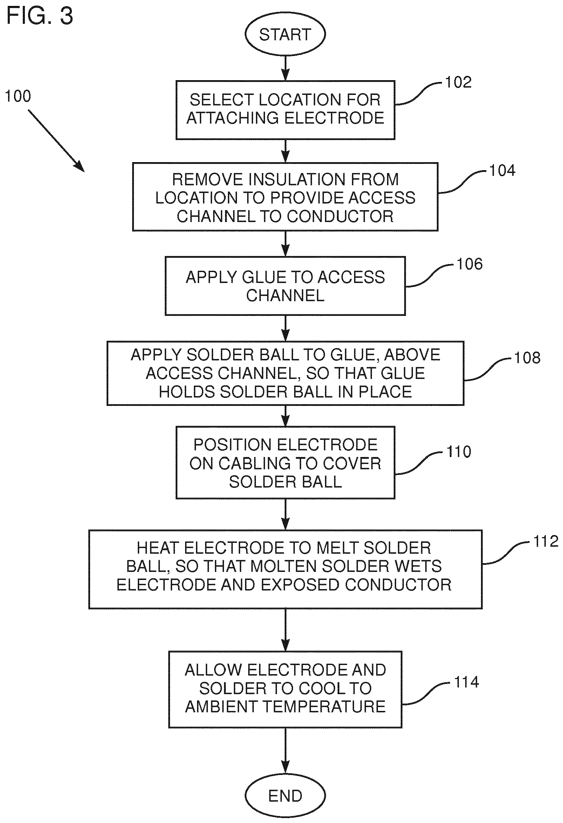

Figs. 2A - 2E are schematic diagrams illustrating steps in attaching electrodes 64 to cabling 10, and Fig. 3 is a flowchart 100 describing the steps, according to embodiments of the present invention. Typically, up to 16 electrodes 64, i.e., electrodes 64A, 64B, ... 640, 64P, may be attached to respective wires of the cabling. The following description refers to a specific electrode by the generic term electrode 64.

Fig. 2A is a schematic top view of the cabling and Figs. 2B - 2E are schematic side views of the cabling in different stages of the attachment of electrode 64. In all views parts of sheath 22 have been cut-away to expose wires 12 of cabling 10, as well as to illustrate the attachment of electrode 64 to the cabling.

Electrode 64 is typically in the form of a conductive ring with dimensions enabling it to be slid over sheath 22. More details of alternative shapes for electrode 64 are provided below. The following explanation assumes that the electrode is to be attached to conductor 14E of colored wire 12E at a distal end of cabling 10.

In an initial step 102 a location for attaching the electrode is selected by first finding colored wire 12E visually, and then finding a required location for the electrode along the cabling. The visual determination is possible since sheath 22 is transparent.

In an access step 104 a section of sheath 22 above the wire, and a corresponding section of insulating layer 16E, are removed. The removal provides an access channel 42, through the insulation of sheath 22 and also through the insulation of layer 16E, to a section 46 of conductor 14E, as is illustrated in Fig. 2A. The removal of the insulation, which exposes the section of conductor 14E, may be by any convenient means, such as by using a laser to penetrate the insulation, or by mechanical removal of the insulation.

In a glue application step 106, glue 44 is applied over the access channel. The application of the glue typically covers channel 42, as is illustrated in Fig. 2B.

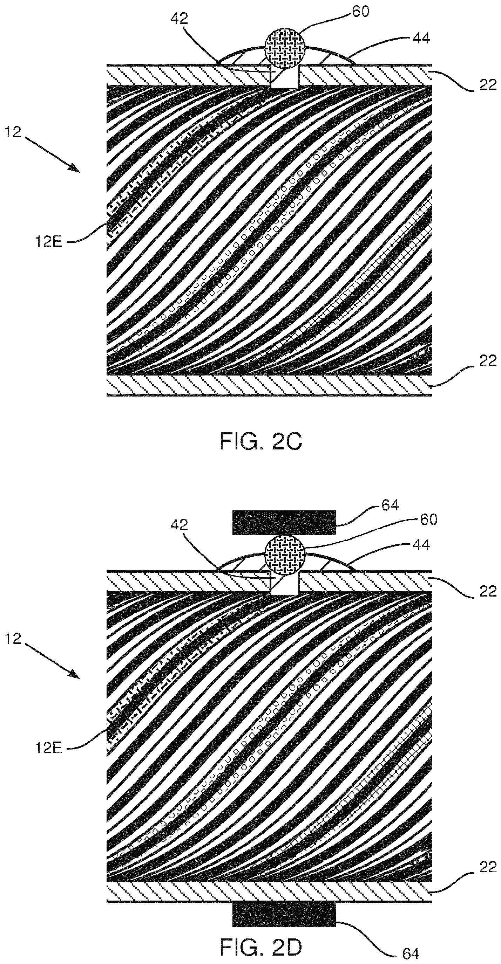

In a solder ball step 108, a solder ball 60 is positioned in proximity to the entrance of access channel 42, and is held in place relative to the channel by glue 44. The positioning of the solder ball is illustrated in Fig. 2C.

Typically, solder ball 60 is formed from a low melting point metal, or a low melting point alloy. In one embodiment of the present invention, solder ball 60 is formed from indium.

In an electrode positioning step 110, electrode 64 is located to be in contact with the solder ball. By way of example electrode 64 is assumed to be in the form of a ring, and is also referred to herein as ring electrode 64. In some embodiments ring electrode 64 completely encircles cabling 10, in which case the positioning of ring electrode 64 may be implemented by sliding the ring along cabling 10 so that the ring covers the solder ball. In alternative embodiments ring electrode 64 partially encircles cabling 10, as a "broken" ring, in which case the positioning of ring electrode 64 may be implemented by pushing the ring over cabling 10 so that the ring covers the solder ball. In the remaining description of the flowchart, ring electrode 64 is assumed to completely encircle the cabling. The positioning of ring electrode 64, for a completely encircling ring, to cover the solder ball is illustrated in Fig. 2D.

In a heating step 112 ring electrode 64 is heated, and while being heated is pressed onto ball 60. The combination of heat and pressure melts the solder of the ball so that the ball deforms, and so that the molten solder wets the inner surface of ring electrode 64 and also penetrates channel 42. The molten solder penetrating the channel wets the exposed section of conductor 14E.

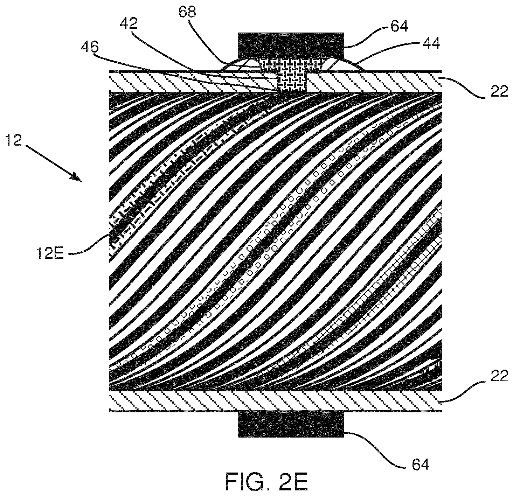

In a final step 114, the heating applied to the ring electrode is removed, and the electrode and solder are allowed to cool, typically to ambient room temperature. On cooling, the solder solidifies into a solid solder bridge 68, as is illustrated in Fig. 2E. Bridge 68, which corresponds to a deformed solder ball, connects electrode 64 and section 46 of conductor 14E.

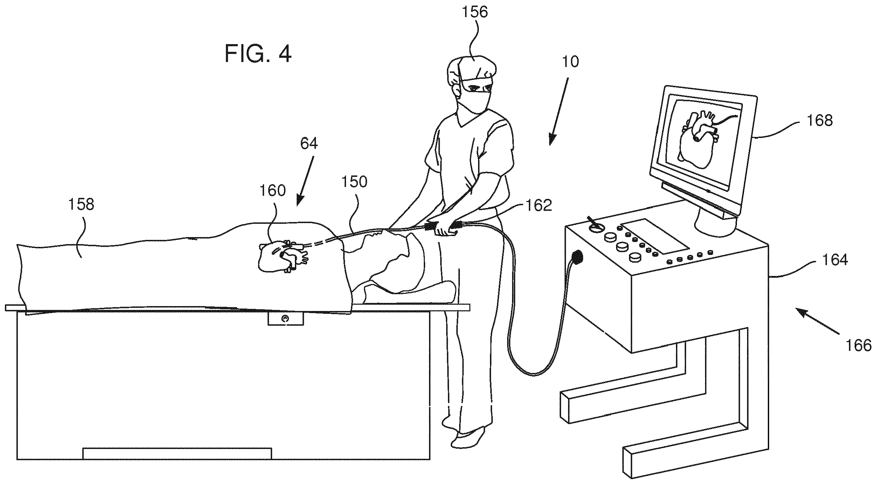

Fig. 4 is a schematic illustration of an invasive medical procedure using cabling 10, according to an embodiment of the present invention. In preparation for the procedure, cabling 10 is incorporated into a catheter 150, the cabling having electrodes 64 at its distal end. Catheter 150 uses one set of cabling 10, and is typically a catheter that is used for measuring electrical properties of a body cavity. At its distal end catheter 150 may be straight or curved as a lasso or helical catheter.

By way of example, a medical professional 156 is assumed to insert catheter 150 into a patient 158 in order to acquire electropotential signals from a heart 160 of the patient. The professional uses a handle 162 attached to the catheter in order to perform the insertion, and signals generated at electrodes 64 are conveyed to a console 164.

Console 164 comprises a processing unit 166 which analyzes the received signals, and which may present results of the analysis on a display 168 attached to the console. The results are typically in the form of a map, numerical displays, and/or graphs derived from the signals.

A more detailed description of the use of a catheter such as catheter 150 in acquiring electropotentials is provided in U.S. Patents 6,584,345 and 6,400,981, both to Govari.

While the description above has been generally directed to attaching an electrode to cabling having wires coiled in a single-handed manner, it will be appreciated that the scope of the present invention includes cabling that is braided, i.e., wherein the cabling comprises wires formed as both left- and right-handed coils. It will also be appreciated that the electrode attached to the cabling need not be in the form of a ring encircling the cabling, but rather may be any convenient shape that does not encircle the cabling.

## Claims

1. A method for attaching an electrode to cabling (10), comprising:
providing a cable comprising a plurality of insulated wires (12) coiled around a central core (18), wherein the wires are covered with a sheath (22), and wherein the sheath (22) is transparent, and an insulating layer of at least one of the plurality of insulated wires (12) is colored differently from the colors of insulating layers of the remaining plurality of insulated wires;
selecting a location for attaching a respective electrode (64) by finding a colored insulated wire visually from the plurality of insulated wires, and then finding a required location for the respective electrode (64) along the cabling;
removing a section of the sheath (22) and removing a corresponding section of insulation (16) from the colored insulated wire (12) in a set of the coiled wires, wherein the section of the sheath is removed from above the wire, so as to provide a respective access channel (42) through the sheath (22) and through the insulation (16) to a respective section of a respective conductor (14) of the wire in the set while the respective section remains coiled on the central core (18) ; and
fastening the respective electrode (64) to the respective access channel (42) while the respective section remains coiled on the central core (18),
wherein the method further comprises positioning a solder ball (60) in proximity to the respective access channel (42) prior to fastening the respective electrode (64), and wherein fastening the respective electrode (64) comprises melting the solder ball (60) so that solidified solder connects the respective section of the respective conductor (14), via the respective access channel (42), to the respective electrode (64).

2. The method according to claim 1, wherein positioning the solder ball (60) comprises applying glue (44) over the respective access channel (42), and holding the solder ball (60) in place with the glue (44).

3. The method according to claim 1, wherein the respective electrode (64) completely encircles the cabling.

4. The method according to claim 1, wherein the respective electrode (64) partially encircles the cabling.

5. The method according to claim 1, wherein the wires (12) are coiled single-handedly around the central core (18) .

6. The method according to claim 1, wherein the set comprises a first group of the wires (12) coiled left-handedly around the central core (18), and a second group of the wires (12) coiled right-handedly around the central core (18).

## Patentansprüche

1. Verfahren zum Befestigen einer Elektrode an Verkabelung (10), umfassend:
Bereitstellen eines Kabels, das eine Vielzahl isolierter Drähte (12) umfasst, die um einen Zentralkern (18) gewickelt sind, wobei die Drähte mit einer Ummantelung (22) bedeckt sind, und wobei die Ummantelung (22) transparent ist, und eine Isolierschicht von mindestens einem von der Vielzahl der isolierten Drähte (12) anders gefärbt ist als die Farben der Isolierschichten der verbleibenden Vielzahl von isolierten Drähten;
Auswählen eines Ortes zum Befestigen einer jeweiligen Elektrode (64), indem ein gefärbter isolierter Draht visuell aus der Vielzahl der isolierten Drähte gesucht wird, und dann Suchen eines erforderlichen Ortes für die jeweilige Elektrode (64) entlang der Verkabelung;
Entfernen eines Stücks der Ummantelung (22), und Entfernen eines entsprechenden Stücks der Isolierung (16) von dem gefärbten Isolierdraht (12) in einem Satz der gewickelten Drähte, wobei das Stück der Ummantelung oberhalb des Drahtes entfernt wird, um so einen jeweiligen Zugangskanal (42) durch die Ummantelung (22) hindurch und durch die Isolierung (16) hindurch zu einem entsprechenden Stück eines jeweiligen Leiters (14) des Drahtes in dem Satz bereitzustellen, während das jeweilige Stück auf dem Zentralkern (18) gewickelt bleibt; und
Anbringen der jeweiligen Elektrode (64) an dem jeweiligen Zugangskanal (42), während das jeweilige Stück auf den Zentralkern (18) gewickelt bleibt,
wobei das Verfahren des Weiteren Positionieren einer Lötkugel (60) in der Nähe des jeweiligen Zugangskanals (42) vor dem Anbringen der jeweiligen Elektrode (64) umfasst, und wobei Anbringen der jeweiligen Elektrode (64) Schmelzen der Lötkugel (60) umfasst, so dass das erstarrte Lot das jeweilige Stück des jeweiligen Leiters (14) über den jeweiligen Zugangskanal (42) mit der jeweiligen Elektrode (64) verbindet.

2. Verfahren nach Anspruch 1, wobei Positionieren der Lötkugel (60) Aufbringen von Kleber (44) über dem jeweiligen Zugangskanal (42) und Halten der Lötkugel (60) in Position mit dem Kleber (44) umfasst.

3. Verfahren nach Anspruch 1, wobei die jeweilige Elektrode (64) die Verkabelung vollständig umringt.

4. Verfahren nach Anspruch 1, wobei die jeweilige Elektrode (64) die Verkabelung teilweise umringt.

5. Verfahren nach Anspruch 1, wobei die Drähte (12) in einer einzigen Richtung gängig um den Zentralkern (18) gewickelt sind.

6. Verfahren nach Anspruch 1, wobei der Satz eine erste Gruppe der Drähte (12) umfasst, die linksgängig um den Zentralkern (18) gewickelt sind, und eine zweite Gruppe der Drähte (12) umfasst, die rechtsgängig um den Zentralkern (18) gewickelt sind.

## Revendications

1. Procédé de fixation d'une électrode à un câblage (10), comprenant :
l'obtention d'un câble comprenant une pluralité de fils isolés (12) enroulés autour d'un noyau central (18), les fils étant recouverts d'une gaine (22), et la gaine (22) étant transparente, et une couche isolante d'au moins un de la pluralité de fils isolés (12) étant colorée différemment par rapport aux couleurs de couches isolantes de la pluralité restante de fils isolés ;
la sélection d'un emplacement pour la fixation d'une électrode respective (64) par localisation visuelle d'un fil isolé coloré parmi la pluralité de fils isolés, puis par localisation d'un emplacement requis pour l'électrode respective (64) le long du câblage ;
le retrait d'une section de la gaine (22) et le retrait d'une section correspondante d'isolation (16) du fil isolé coloré (12) dans un ensemble des fils enroulés, la section de la gaine étant retirée du dessus du fil, de manière à obtenir un canal d'accès respectif (42) à travers la gaine (22) et à travers l'isolation (16) jusqu'à une section respective d'un conducteur respectif (14) du fil dans l'ensemble pendant que la section respective reste enroulée sur le noyau central (18) ; et
la fixation de l'électrode respective (64) au canal d'accès respectif (42) pendant que la section respective reste enroulée sur le noyau central (18),
le procédé comprenant en outre le positionnement d'une bille de brasure (60) à proximité du canal d'accès respectif (42) avant la fixation de l'électrode respective (64), et la fixation de l'électrode respective (64) comprenant la fusion de la bille de brasure (60) de telle sorte qu'une brasure solidifiée relie la section respective du conducteur respectif (14), par l'intermédiaire du canal d'accès respectif (42), à l'électrode respective (64).

2. Procédé selon la revendication 1, dans lequel le positionnement de la bille de brasure (60) comprend l'application de colle (44) sur le canal d'accès respectif (42), et le maintien de la bille de brasure (60) en place avec la colle (44).

3. Procédé selon la revendication 1, dans lequel l'électrode respective (64) encercle entièrement le câblage.

4. Procédé selon la revendication 1, dans lequel l'électrode respective (64) encercle partiellement le câblage.

5. Procédé selon la revendication 1, dans lequel les fils (12) sont enroulés dans un seul sens autour du noyau central (18).

6. Procédé selon la revendication 1, dans lequel l'ensemble comprend un premier groupe des fils (12) enroulés vers la gauche autour du noyau central (18), et un deuxième groupe des fils (12) enroulés vers la droite autour du noyau central (18).
